# EUROPEAN PATENT SPECIFICATION

(11) **EP 1 504 086 B1**
(45) Date of publication and mention of the grant of the patent: **14.05.2008**
(21) Application number: 03725376.2
(22) Date of filing: 08.05.2003
(51) Int. Cl.: C12N 5/00

(54) **CONTROL OF ES CELL SELF RENEWAL AND LINEAGE SPECIFICATION, AND MEDIUM THEREFOR**
KONTROLLE DER SELBSTERNEUERUNG EMBRYONALER STAMMZELLEN, DEREN DIFFERENZIERUNG UND KULTURMEDIUM DAFÜR
REGULATION DE L'AUTORENOUVELLEMENT DE CELLULES SOUCHES EMBRYONNAIRES (ES) ET SPECIFICATION DE LIGNAGE, ET SUBSTANCE APPROPRIEE

(30) Priority: 08.05.2002 GB 0210539
(43) Date of publication of application: 09.02.2005
(73) Proprietor: THE UNIVERSITY COURT OF THE UNIVERSITY OF EDINBURGH, Edinburgh EH8 9YL (GB)
(72) Inventor: SMITH, Austin, G. Institute of Stem Cell Research, West Main Road Edinburgh EH9 3JQ (GB); YING, Qi-Long Institute of Stem Cell Research, West Main Road Edinburgh EH9 3JQ (GB)
(74) Representative: Schlich, George William
(86) International application number: PCT/GB2003/001967
(87) International publication number: WO 2003/095628

(56) References cited:
- JOHANSSON BRITT M ET AL: "Evidence for involvement of activin A and bone morphogenetic protein 4 in mammalian mesoderm and hematopoietic development." MOLECULAR AND CELLULAR BIOLOGY, vol. 15, no. 1, 1995, pages 141-151, XP002255401 ISSN: 0270-7306
- ATSUMI T ET AL: "THE ACTIVIN A-DEPENDENT PROLIFERATION OF PCC3/A/1 EMBRYONAL CARCINOMA CELLS IN SERUM-FREE MEDIUM" DEVELOPMENT GROWTH AND DIFFERENTIATION, JAPANESE SOCIETY OF DEVELOPMENTAL BIOLOGISTS,, JP, vol. 35, no. 1, 1993, pages 81-87, XP001015818 ISSN: 0012-1592
- RAMSFJELL VESLEMOY ET AL: "Distinct requirements for optimal growth and in vitro expansion of human CD34+CD38- bone marrow long-term culture-initiating cells (LTC-IC), extended LTC-IC, and murine in vivo long-term reconstituting stem cells." BLOOD, vol. 94, no. 12, 15 December 1999 (1999-12-15), pages 4093-4102, XP002255402 ISSN: 0006-4971
- MATSUI HISANORI ET AL: "Effect of TGF-beta signaling on ES cells self-renewal." DEVELOPMENT GROWTH & DIFFERENTIATION, vol. 43, no. Supplement, July 2001 (2001-07), page S140, XP009017001 14th International Congress of Developmental biology;Kyoto, Japan; July 08-12, 2001 ISSN: 0012-1592

## Description

The present invention relates to culture conditions and methods of culturing pluripotent stem cells in order to promote stem cell self renewal and to prevent or control differentiation of the stem cells. The invention further provides methods for isolating and maintaining homogeneous preparations of pluripotent stem cells. The methods and compositions provided are suitable for culturing and isolating pluripotent stem cells such as embryonic stem (ES) cells, provided the cells are not derived from human embryos.

The establishment and maintenance of in vitro pluripotent stem cell cultures in the presence of medium containing serum and Leukaemia Inhibitory Factor (LIF) is well known (Smith et al. (1988) Nature 336: 688-90). Such methods have been used to maintain pluripotent embryonic stem (ES) cells from strains of permissive mice over many passages. Maintenance and self renewal of pluripotent stem cell cultures is further supported where the stem cells are cultured in the presence of feeder cells or extracts thereof, usually mouse fibroblast cells. Under such conditions it is possible to maintain human ES cells in a pluripotent state over many passages in culture.

A continuing problem in this field is that, despite intense efforts, it remains the case that pluripotent cultures of ES cells can be derived and maintained for extended periods only from a few species and even in those species not from all embryos. In some cases, pluripotent cells can be identified but can not then be maintained in culture for sufficient time to enable study of the cells or their genetic manipulation. This is particularly the case for rodent (other than some strains of mice) cells.

A further problem is that ES cells that can indeed be maintained in a pluripotent state in culture over many passages can only be so maintained using medium that contains serum or serum extract, and hence is undefined, or using cell culture conditions that require the presence of other cells, such as the fibroblast feeder cells used to maintain human ES cells. However, where ES cells are intended to be subjected to subsequent controlled differentiation into desired cell types, it is undesirable to utilise an undefined culture medium or to have heterologous cells present.

The serum typically used in culturing pluripotent stem cells is fetal calf (bovine) serum, which is known to contain a complex mixture of cytokines and other signalling molecules. In order to control differentiation pathways it is undesirable to introduce unknown cytokines to the culture medium whose influence on the eventual outcome of differentiation is unquantifiable, and could be potentially deleterious. Further, each serum batch is unique and introduces variation into culture protocols.

As a result, the ES cells obtained by culture in such complex media, and any differentiated progeny thereof, risk being contaminated by components of the media and/or by cells such as feeder cells that are required to maintain the ES cells. These factors mitigate against development of good manufacturing practices for therapeutic and other applications of ES cells and their progeny.

When deriving a differentiated cell population from an ES cell culture, it is desirable to be able to convert a high proportion of the ES cells into progeny of the same type - i.e. to maintain as homogeneous a population of cells as possible. However, in practice, it is observed that, following differentiation, a cell population is obtained that contains a heterogenous mixture of cells. Hence, it is desirable to be able to carry out differentiation of an ES cell population obtaining a purer population of progeny.

EP 1077254 describes methods and compositions for the differentiation of stromal cells from adipose tissue, which may include interleukins, FGF and serum, and amounts of TGF-β sufficient to induce differentiation into smooth muscle.

EP 0753574 describes methods and compositions for ex *vivo* human progenitor cell expansion. The culture medium contains stromal cells, typically transformed fibroblast cells.

WO 00/05344 describes maintenance of *Drosophila* germline stem cells and propagation of somatic stem cells of other species when co-cultured with genetically engineered *Drosophila* cells.

WO 96/40866 describes serum-free culture of human haematopoietic progenitor and stem cells in a culture medium containing at least one of a peptone, a protease inhibitor and a pituitary extract.

US 2002/0028510 describes methods and compositions for the differentiation of pluripotent cells from umbilical cord blood into neuronal cell types.

US 5750376 describes methods and compositions for differentiation of multipotent neural stem cells in culture medium supplemented with at least one growth factor.

Wiles and Johansson, Exp. Cell Research, 1999 (247) pgs 241-248 describes maintenance of undifferentiated cell lines in the presence of LIF and Fetal Bovine Serum. When ES cells were grown in a serum free medium they rapidly lost their ES cell phenotype and developed into a range of cell types, including neuroectoderm.

Hence, an object of the invention is to provide methods of culturing and culture media suitable for pluripotent stem cells, which are capable of supporting self-renewal of said stem cells in an undifferentiated state for many passages, provided the cells are not derived from human embryos. A further object of the invention is to provide a culturing system that permits maintenance of a pluripotent stem cell culture *in vitro* until differentiation of the cells is induced in a controlled manner, provided the cells are not derived from human embryos. A still further object of the invention is to provide methods and compositions that enhance the isolation of pluripotent stem cells and facilitate their isolation from organisms refractory to ES cell isolation or from which pluripotent stem cells have not yet been isolated.

5The present invention is based on the observation that culturing pluripotent stem cells, such as ES cells, in serum-free media comprising agonists of the gp130 (e.g. LIF) and bone morphogenic protein (BMP) family (e.g. BMP4) signalling pathways promotes self renewal of the stem cells for multiple passages. Hence, in the presence of gp130 signalling, an agonist of the BMP family signalling pathway provides a self renewal stimulus rather than a pro-differentiation signal.

Accordingly, a first aspect of the invention provides the use of a combination of:-
(a) a bone morphogenic protein (BMP); and
   a cytokine acting through gp130 in promoting self-renewal of pluripotent cells in culture, wherein the pluripotent cells are not of human embryonic origin.

One or more signalling pathways downstream from a BMP receptor can be activated using a polypeptide agonist of a BMP receptor.

Activation of one or more gp130 downstream signalling pathways can be achieved by use of a cytokine acting through gp130, for example a cytokine or other agonist of the LIF receptor.

An agonist of a BMP receptor is preferably a bone morphogenetic protein (BMP) such as BMP-4. Known homologues of BMP4, such as BMP2 and BMP7, are also suitable, as are homologues from non-mammalian species such as decapentaplegic (dpp) from *Drosophila melanogaster.* Good results have been obtained using BMPs 4, 8 and 2. Good results have also been obtained using a chimera of BMP4 and BMP8, which is easier to obtain in secreted form than BMP4. The term "agonist" is also intended to embrace mimetics, fusion proteins or chimaeras of BMP signalling polypeptides, and fragments, variants and derivatives thereof, capable of activating BMP receptors.

Cytokines capable of acting through gp130, and thus of activating gp130 signal transduction, include LIF, CNTF, cardiotrophin, oncostatin M and IL-6 plus sIL-6 receptor. Suitable cytokines include mimetics, fusion proteins or chimaeras that can bind to and/or activate signalling though gp130.

The role of cytokines acting through gp130 in the presence of serum is well established, but the capacity of those cytokines to sustain undifferentiated cells in the absence of serum is limited.

Previous data on BMPs, both from developmental studies in vivo and in ES cells, points to activity in driving mesodermal and epidermal differentiation. There is no evidence in the literature that activation of the BMP signalling pathway can enhance self-renewal in pluripotent stem cells.

Chemically defined serum-free media for ES cell differentiation comprising LIF and BMP4 has been previously disclosed by Johansson and Wiles (Mol. Cell. Biol. (1995) 15(1): 141-51). However, the medium also contained an undefined albumin component and both LIF and BMP4 were only combined in a culture medium used for promoting differentiation of ES cells into mesoderm and haematopoietic cells. Differentiation into cells expressing the mesodermal marker gene, *Brachyury,* was observed after 5 days in culture (see page 145, first paragraph). In the absence of BMP4, the chemically defined medium supplemented with much higher concentrations of LIF is capable of maintaining ES cells for a maximum of three passages (see page 143, second column). Hence, Johansson and Wiles describe a composition which promotes differention induced by formation of multicellular aggregates and differentiation of ES cells, not self renewal of ES cells.

The present invention therefore provides, in one embodiment, culture of ES cells in medium that may be free of serum, serum extract, feeder cells and feeder cell extract, provided the cells are not derived from human embryos.

When using the LIF and BMP4 -containing medium of a specific embodiment of the present invention extended passaging of ES cells is possible. In one example, murine ES cells have been maintained for more than 20 passages over a three month period. Cells were passaged every 2-4 days depending on the plating cell density, though occasionally cells were passaged 7-10 days after plating at low density.

Another advantage of the present culture system is that differentiation of ES cells is reduced compared to culture in the presence of serum. This is significant because often the most pluripotent ES cells tend to differentiate considerably in serum, making their manipulation and expansion problematic.

ES cells in the cultures were examined and found still to maintain ES cell morphology and to express ES cell markers (positive staining for alkaline phosphatase). When using a GFP reporter gene under the control of the ES cell specific Oct-4 promoter green fluorescence was seen in the ES cells. Similarly, β-galactosidase expression from the Sox2 promoter was maintained. ES cell mRNA markers such as Oct4, Nanog, Rex1 and Sox2 are expressed, further confirming the cells are ES cells. The morphological differentiation of cells in the culture is found to be very low, and lower than a comparative culture of ES cells in serum-containing medium. Thus, the culture conditions of the present invention enable ES cells to self-renew in the absence of serum.

Embryonic stem cells have been reported from a number of mammalian sources including mouse (Bradley et al (1984) Nature 309: 255-56), American mink (Mol Reprod Dev (1992) Dec;33(4):418-31), pig and sheep (J Reprod Fertil Suppl (1991);43:255-60), hamster (Dev Biol (1988) May;127(1):224-7) and cow (Roux Arch Dev Biol (1992); 201: 134-141). It will be appreciated that the methods and compositions of the present invention are suitable for adaptation to culturing of other mammalian pluripotent cell cultures, including human, primate and rodent, and avian ES cells, provided the cells are not derived from human embryos.

Specifically, with regard to human ES cells, it is known that human ES cells respond to LIF and therefore the medium and methods disclosed herein, in which a self-renewal stimulus is obtained in response to a combination of LIF and BMP4, are of application to human ES cells.

Suitable cell densities for the methods of the invention will vary according to the pluripotent stem cells being used and the natures of any desired progeny. Good results have been obtained by culturing embryonic stem cells in monolayer culture, dissociating the embryonic stem cells and subsequently culturing the embryonic stem cells in monolayer culture on a culture surface at a density of from 0.2 - 2.5 x 10⁴ cells per cm², more particularly at a density of from 0.5 - 1.5 x 10⁴ per cm². The cells proliferate as adherent monolayers and are observed to have a doubling time comparable to ES cells grown in serum-containing media together with LIF.

Typical surfaces for culture of ES cells and their progeny according to the invention are culture surfaces recognized in this field as useful for cell culture, and these include surfaces of plastics, metal, composites, though commonly a surface such as a plastic tissue culture plate, widely commercially available, is used. Such plates are often a few centimetres in diameter. For scale up, this type of plate can be used at much larger diameters and many repeats plate units used.

It is further common for the culture surface to comprise a cell adhesion protein, usually coated onto the surface. Receptors or other molecules on the cells bind to the protein or other cell culture substrate and this promotes adhesion to the surface and it is suggested promotes growth. Gelatin coated plates are commonly available and are suitable for the invention, and other proteins may also be used.

Including inhibitor of the FGF receptor in an amount sufficient to suppress the differentiation of the ES cells in the culture medium for at least part of the culturing period is found to suppress the tendency of ES cells to differentiate. The ES cells are cultured in defined serum-free media comprising LIF and an FGF receptor inhibitor for a specified period before the FGF receptor inhibitor is removed and replaced by an agonist of the BMP signalling pathway (e.g. BMP4). Suitable FGF receptor inhibitors include the compounds SU5402 and PD173074. Alternatively, a competitive inhibitor of the FGF receptor can be used, suitably a soluble form of the receptor.

It is an option not to remove the FGF receptor inhibitor. Hence, the FGF receptor inhibitor is present in the culture medium for an extended period, either in the presence or absence of an agonist of the BMP signalling pathway. ES cells can be grown in culture for at least 20 passages in N2B27 medium in the presence of LIF and an FGF inhibitor, in the absence of BMP. If the FGF receptor inhibitor is not removed from the medium, it is preferred that it is a specific inhibitor and has little or no activity on other receptors.

A second aspect of the invention provides a method of culture of non-human ES cells so as to promote ES cell self renewal, comprising maintaining the ES cells in medium containing:-
(a) a BMP; and
(b) a cytokine acting through gp130.

Methods disclosed herein can be used for stimulating self-renewal of ES cells in medium which is free of serum and free of serum extract, which cells have previously been passaged in the presence of serum or serum extract. Preferably, such methods are also carried out in the absence of feeder cells and/or feeder cell extracts. For example, culture of non-human ES cells can be carried out comprising the steps of:-
maintaining the ES cells in a pluripotent state in culture, optionally on feeders, in the presence of a cytokine acting though gp130 and serum or an extract of serum;
passaging the ES cells at least once;
withdrawing the serum or the serum extract from the medium and withdrawing the feeders (if present), so that the medium is free of feeders, serum and serum extract; and
subsequently maintaining ES cells in a pluripotent state in the presence of a polypeptide agonist of a BMP receptor and a cytokine acting through gp130.

At around the time that the serum or extract of serum is withdrawn from the medium, it is an option to add to the medium an FGF receptor inhibitor in an amount sufficient to suppress differentiation of the ES cells. It is an option for the FGF receptor inhibitor to be withdrawn at the same time as or subsequent to maintenance of the cells in the presence of a polypeptide agonist of a BMP receptor.

The present invention also provides a method of obtaining a transfected population of non-human ES cells, comprising:-
transfecting ES cells with a construct encoding a selectable marker;
plating the ES cells;
culturing the ES cells in the presence of
   (a) a BMP; and
   (b) a cytokine acting through gp130; and selecting for ES cells that express the selectable marker.

The selectable marker may encode antibiotic resistance, a cell surface marker or another selectable marker as described e.g. in EP-A-0695351.

In a further embodiment, the present invention provides a method of culture of non-human ES cells, comprising the steps of transferring an individual ES cell to a culture vessel, such as an individual well on a plate, and culturing the ES cell in the presence of a BMP and a cytokine acting through gp130 so as to obtain a clonal population of ES cells, all of which are progeny of a single ES cell.

Once a stable, homogenous culture of ES cells is obtained, the culture conditions can be altered to direct differentiation of the cells into one or more cell types selected from ectodermal, mesodermal or endodermal cell fates. Addition of, or withdrawal of cytokines and signalling factors, can enable the derivation of specific differentiated cell populations at high efficiency. Differentiation of an ES cell towards a non-neuroectodermal fate may be achieved by maintaining the ES cell in the presence of a cytokine acting through gp130 and a polypeptide agonist of the BMP receptor and then withdrawing the cytokine whilst maintaining the BMP receptor agonist and/or adding a further signalling molecule capable of directing differentiation.

For example, exposure to BMP4 in the absence of LIF leads to induction of mesendodermal cell types. Withdrawal of cytokines acting through gp130 and polypeptide agonists of a BMP receptor and/or blockade of both pathways leads to induction of a neurectodermal phenotype. Alternatively, other signalling factors can be added to the culture conditions to direct other differentiation pathways - for example, activin, sonic hedgehog (shh), Wnts and FGFs.

In use, towards the end of ES cell culture, it is desirable to remove BMP4 at least one passage before differentiation is initiated, in order to ensure that the BMP signal declines and there is no legacy of the BMP signal during subsequent differentiation. In one embodiment, an FGF receptor antagonist is added to the cultures for one to two passages whilst removing BMP from the cultures.

Further aspects of the invention provide for cell culture media for self-renewal of ES cells. One such medium comprises:-
basal medium;
a cytokine acting through gp130;
an iron-transporter;
wherein the medium is free of serum and serum extract.

Basal medium is medium that supplies essential sources of carbon and/or vitamins and/or minerals for the ES cells. The basal medium is generally free of protein and incapable on its own of supporting self-renewal of ES cells. The iron transporter provides a source of iron or provides ability to take up iron from the culture medium. Suitable iron transporters include transferrin and apotransferrin.

It is preferred that the medium further comprises insulin or insulin-like growth factor and is free of feeder cells and feeder cell extract.

The invention also provides cell culture media comprising:-
(a) a BMP; and
(b) a cytokine acting through gp130.

The culture medium is optionally supplemented with an inhibitor of an FGF receptor in an amount sufficient to suppress differentiation of ES cells, or, when differentiation is desired, signalling factors that direct differentiation of ES cells toward a specific phenotype.

It is preferred that the medium is free of serum or serum extract. Most preferably, the medium is fully defined.

In a preferred embodiment of the invention the culture medium comprises the gp130 receptor binding cytokine, LIF, at a concentration of between 10U/ml and 1000U/ml, more preferably between 50U/ml and 500U/ml, even more preferably in the region of 100 U/ml.

It will be appreciated by a person of skill in the art that activation of signalling pathways downstream from a BMP receptor can be effected by either upstream agonists of the receptor (e.g. receptor ligands), constitutively active receptors, or activated downstream components of the signalling pathway, for example the SMAD signal transduction molecules. Likewise upstream effectors (eg. cytokines) and downstream effectors (eg. Stats) of the gp130 signal transduction pathway are capable of activating this pathway also. Thus, compositions comprising molecules capable of activating BMP receptor signalling pathways and gp130 signalling pathways in order to promote self renewal of pluripotent stem cells are also disclosed herein.

It is further preferred, according to the invention, that culture of cells is carried out in an adherent culture, and in examples of the invention it has been found that following maintenance of cells in a pluripotent state, differentiation can be induced with a high degree of uniformity and with high cell viability. Adherent cultures may be promoted by the inclusion of a cell adhesion protein, and in specific examples of the invention gelatin has been used as a coating for the culture substrate.

It is also preferred to culture pluripotent cells according to the invention in monolayer culture, though it is optional for cells to be grown in suspension culture or as pre-cell aggregates; cells can also be grown on beads or on other suitable scaffolds such as membranes or other 3-dimensional structures.

A further component of medium for culture of pluripotent cells according to the invention, and which is preferred to be present, is a factor promoting survival and/or metabolism of the cells. In a specific embodiment of the invention, cells are cultured in the presence of insulin. An alternative factor is insulin-like growth factor and other such survival and/or metabolism promoting factors may alternatively be used.

Culture medium used in the examples of the invention preferably also comprises serum albumin. This can be used in purified or recombinant form, and if in a recombinant form this has the advantage of absence of potential contaminating factors, cytokines etc. The culture medium does not need to contain serum albumin and this component can be omitted or replaced by another bulk protein or by a synthetic polymer (polyvinyl alcohol) as described by Wiles et al.

A particularly preferred medium of the invention is one that is fully defined. This medium does not contain any components which are undefined, that is to say components whose content is unknown or which may contain undefined or varying factors that are unspecified. An advantage of using a fully defined medium is that efficient and consistent protocols for culture and subsequent manipulation of pluripotent cells can be derived. Further, it is found that maintenance of cells in a pluripotent state is achievable with higher efficiency and greater predictability and that when differentiation is induced in cells cultured using a defined medium the response to the differentiation signal is more homogenous then when undefined medium is used.

Methods of the invention also include a method of obtaining a differentiated cell comprising culturing a pluripotent cell as described and allowing or causing the cell to differentiate, wherein the cell contains a selectable marker which is capable of differential expression in the desired differentiated cell compared with other cell-types, including pluripotent stem cells, whereby differential expression of the selectable marker results in preferential isolation and/or survival and/or division of the desired differentiated cells, provided the cells are not derived from human embryos.

The differentiated cell can be a tissue stem or progenitor cell, and may be a terminally differentiated cell.

Also disclosed herein is a method of isolating a pluripotent stem cell or an EG or EC cell comprising culturing tissue from an embryo, fetus or adult in medium containing:-
(a) a cytokine acting through gp130; and
(b) a BMP; and/or
(c) an inhibitor of a FGF receptor, provided the cells are not derived from human embryos.

Preferably, the medium is a fully defined medium.

Also disclosed is the use of a combination of:-
(a) an inhibitor of FGF receptor signalling; and
(b) an activator of gp130 downstream signalling pathways in promoting self-renewal of pluripotent cells in culture.

The methods and compositions of the invention are illustrated in the accompanying drawing in which:-
Fig. 1 shows a light microscopy image of ES cells grown at either high or low cell density in the serum-free defined media of the invention; cells expressing the GFP protein under the control of the ES cell specific promoter, *Oct4*, fluoresce;
Fig. 2 shows light microscopy images of the ES cells stably transfected with pPCAG-tauGFP-IP in serum-free medium in the presence of LIF and BMP4;
Fig. 3 shows a chimeric mouse embryo (at embryonic day 11) created from blastocysts injected with GFP-expressing ES cells of Fig. 2;
Fig. 4 shows phase contrast and fluorescence images of individual ES cells plated at low density in the presence of LIF and BMP4, and clonal populations derived therefrom at 6 days post-plating;
Fig. 5 shows OS25 and Oct4GFP ES cells (passage 3) grown in DMEM F12 plus neurobasal medium (ratio 1:1) supplemented with IGF-1 or insulin, apotransferrin, progesterone, putrescine and sodium selenite;
Fig. 6 shows an immunoblot demonstrating phosphorylation of p-STAT3, STAT3, p-Smad1 and p-Smad2 at 15 min and 1 hour post administration of one or more of LIF/BMP4/TGF-β1/serum to ES cells; and
Fig. 7 shows an immunoblot demonstrating phosphorylation of ERKs, p38, Smads1 and 2 and STAT-3 at 0, 15 mins, 30 mins, 1, 2, 4, 8 and 24 hours post administration of LIF plus BMP4 plus TGF-β1 to ES cells. The phosphorylation pattern at 1 hour post-administration is compared with that 1 hour post-administration of LIF + serum.

The invention is demonstrated in use by the following examples.

### EXAMPLES

### Example 1. Method for growing ES cells in serum free, feeder cell free defined culture media

- N2B27: (1:1 mixture of DMEM/F12 medium supplemented with modified N2 (25µg/ml insulin, 100µg/ml apo-transferrin, 6ng/ml progesterone, 16µg/ml putrescine, 30nM sodium selenite and 50µg/ml bovine serum albumin) and Neurobasal medium supplemented with B27)

ES cells were cultured in 0.1% gelatin coated dishes in N2B27 medium supplemented with LIF (100U/ml) and BMP4 (10ng/ml). For passaging, a standard protein-free cell dissociation buffer was used to dissociate cells.

The plating density of the cells was approximately 1-5 x 10⁴ /cm².

At the start of culture, the medium was further supplemented with SU5402 (5µM) to suppress differentiation. Cells were transferred to media free of SU5402 after two passages.

ES cells were maintained in these serum free conditions for 20 passages over a three month period. Cells were normally passaged every 2-4 days depending on plating density. Occasionally, cell were passaged 7-10 days after plating at low clonal density.

The ES cells maintained pluripotency after multiple passages. Upon withdrawal of LIF and BMP4 the ES cells differentiated into *Sox1* expressing neural precursor cells. In the presence of BMP4 but absence of LIF, the ES cells differentiated into large flat cells of characteristic morphology.

### Example 2. Oct4-GFP expression is maintained in ES cells cultured under serum free conditions

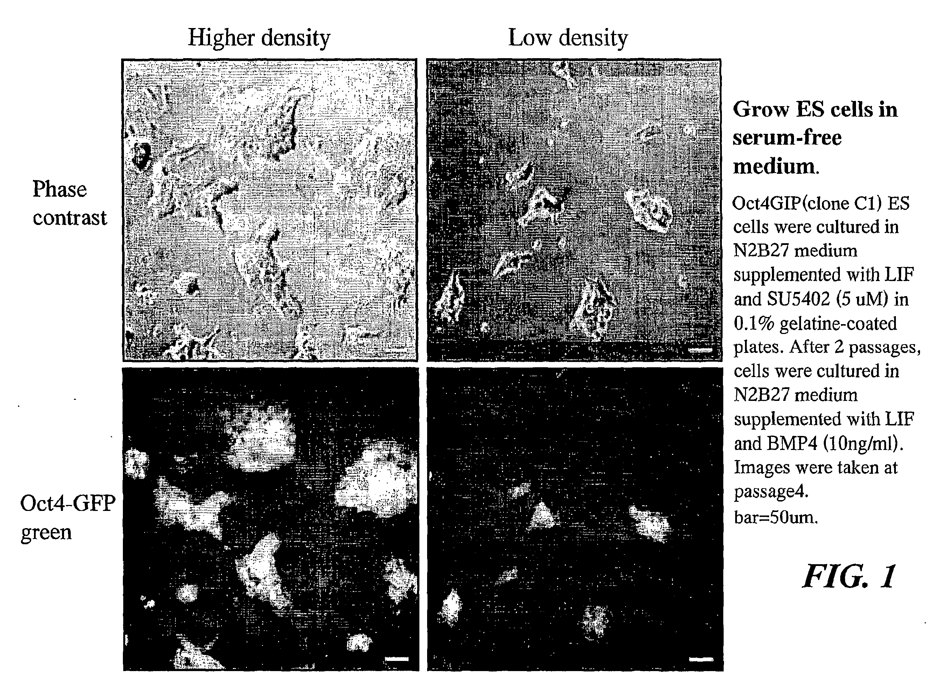

Oct4GFP (clone C1) ES cells were cultured in N2B27 medium supplemented with LIF and SU5402 (see Example 1) in 0.1% gelatine coated plates. After two passages cells were cultured in N2B27 medium supplemented with LIF and BMP4. After a further two passages light microscope images were taken of the cells under phase contrast to show morphology and UV fluorescence to show expression of GFP. The microscopy images are shown in Fig. 1.

It is apparent that both the morphology and expression of GFP indicates that after four passages in serum free media the ES cells maintained their pluripotent phenotype.

### Example 3. Stable transfection of ES cells

E14 TG2A ES cells were cultured in DMEM F12 plus neurobasal medium supplemented with N2-B27 additives and also supplemented with LIF and BMP4. The cells were propagated on 0.1% gelatin coated plates, harvested and electroporated with pPCAG-tauGFP-IP. Transfected cells were replated on a 10cm diameter dish at a density of 10⁵-10⁶ per dish. After 24 hours, 0.5µg/ml puromycin was added to select for positive colonies.

Between 8 and 10 days later, single GFP positive colonies were picked into each single well of a 96 well plate and the cells cutured in the same medium as described above.

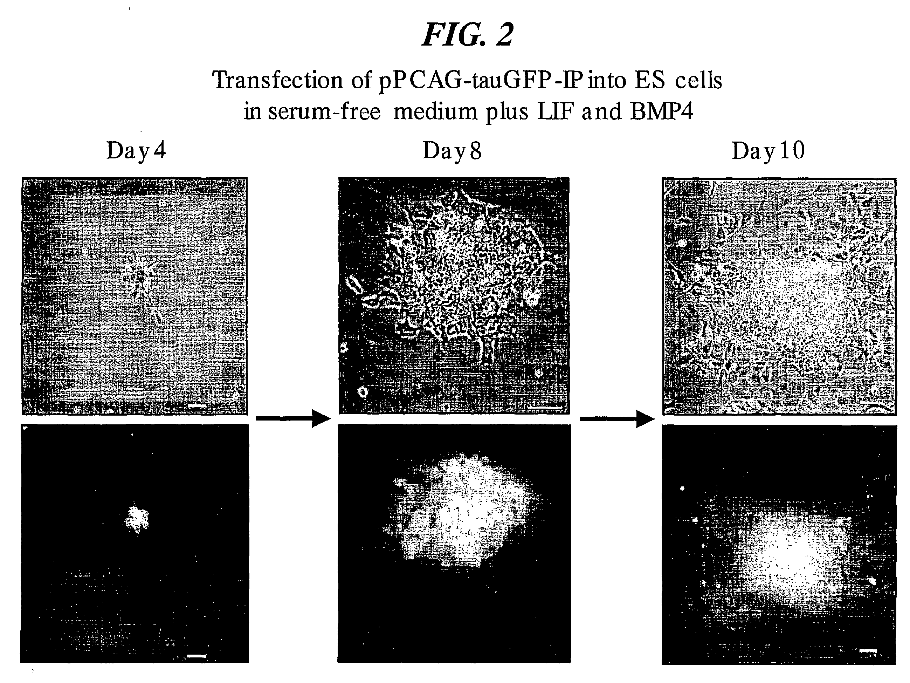

Stable transfection of the ES cells by GFP fluorescence and expansion of morphologically undifferentiated ES cells was observed, as demonstrated in Fig. 2.

### Example 4. Chimeric mouse

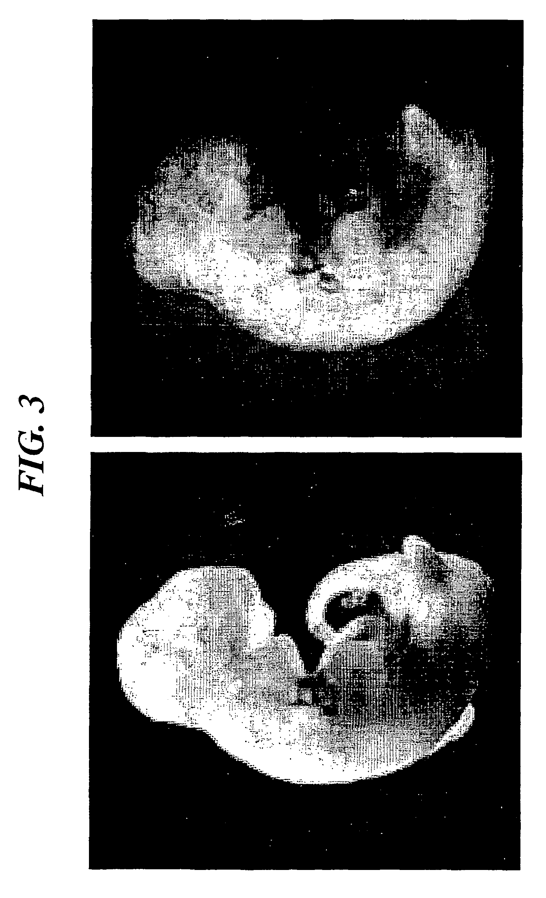

GFP expressing ES cells obtained as described in Example 3 above were injected into blastocysts. GFP expressing ES cells contributed to a wide range of cell-types in a chimeric mouse embryo, as shown in Fig. 3 (embryonic day 11). Live bom chimeric mice were obtained, having a chimeric coat, confirming that the cells were true ES cells.

### Example 5. Clonal self-renewal of ES cells

Individual ES cells were picked and transferred into wells of a 96 well plate, and cultured in medium as described for Example 3 above.

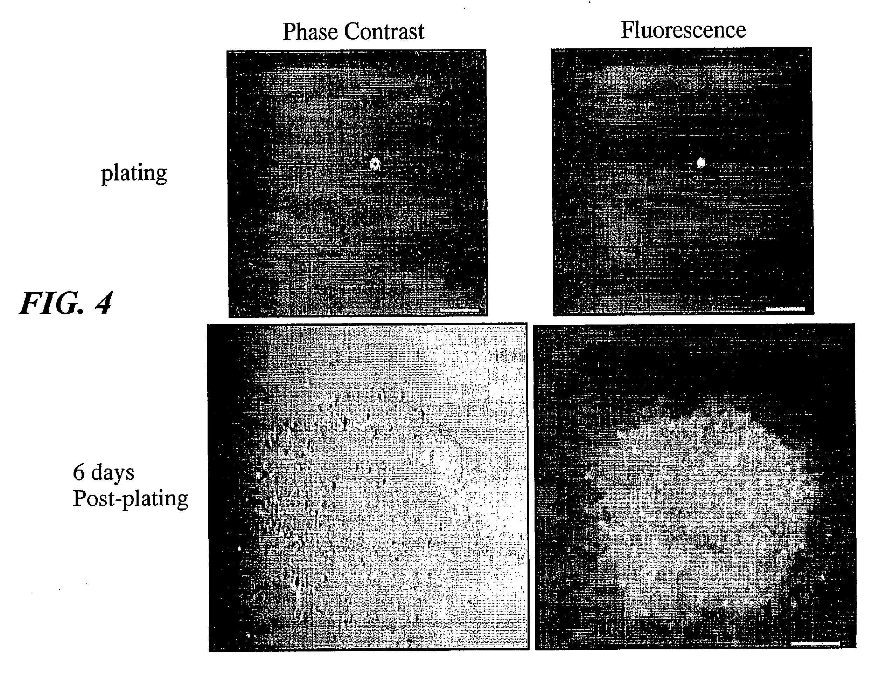

We found the efficiency of cloning of these ES cells, previously grown in serum -free media for at least one passage, to be similar to that obtainable using serum-containing medium, i.e. about 10% efficiency. The clones grew and could be passaged and grown further as undifferentiated ES cells in the presence of LIF and BMP4, as shown in Fig. 4.

In previous experiments (data not published) we have discovered that ES cells grown in serum-containing medium when transferred directly to a serum-free medium demonstrate lower formation of clonal colonies.

### Example 6. Growth of ES cells in fully defined medium

ES cells were grown in a fully defined, albumin free, medium comprising DMEM F12 plus neurobasal medium (ratio 1:1) supplemented with IGF-1 10µg/ml (or insulin at 5-25µg/ml), apotransferrin 100µg/ml, progesterone 6µg/ml, putrescine 16µg/ml and sodium selenite 30nmol.

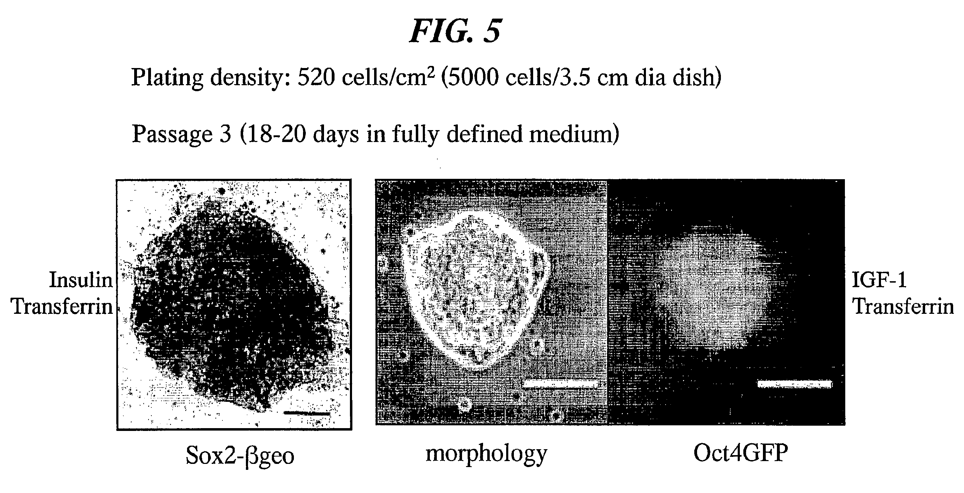

Oct4GFP ES cells were passaged 6 times (cells passaged every 6-8 days) using cell dissociation buffer and replated after each passage at low density. Microscopy images taken after passage 3 are shown in Fig. 5.

### Example 7. Use of serum-free medium and transient growth factor stimulation

ES cells were grown initially in a medium comprising DMEM F12 plus neurobasal medium (ratio 1:1) supplemented with N2-B27. ES cells were plated at very low density, about 1000-10,000 cells on a 3.5cm diameter plate and grown on in the same medium supplemented with LIF and BMP4.

Intermittently, TGFβ at 1-2ng/ml or activin A at 5ng/ml were added to the culture medium and the cells grown on again.

We observed enhanced numbers of undifferentiated ES cells, indicating increased proliferation or increased ES cell survival, or both. Hence the rank order of efficiency was found to be:-
LIF/ BMP4/ activin A (5 ng/ml) > LIF/ BMP4/ TGF-β (1-2 ng/ml) > LIF/ activin A > LIF/ BMP4 > LIF/ TGF-β 1> LIF only.

After more (5-6) passages the rank order of efficacy changed to:-
LIF/ BMP4 > LIF/ BMP4/ activin A > LIF/ BMP4/TGF-β > LIF /activin A= LIF only.

When either TGFβ or activin A was continuously present, progressive differentiation of the ES cells was observed.

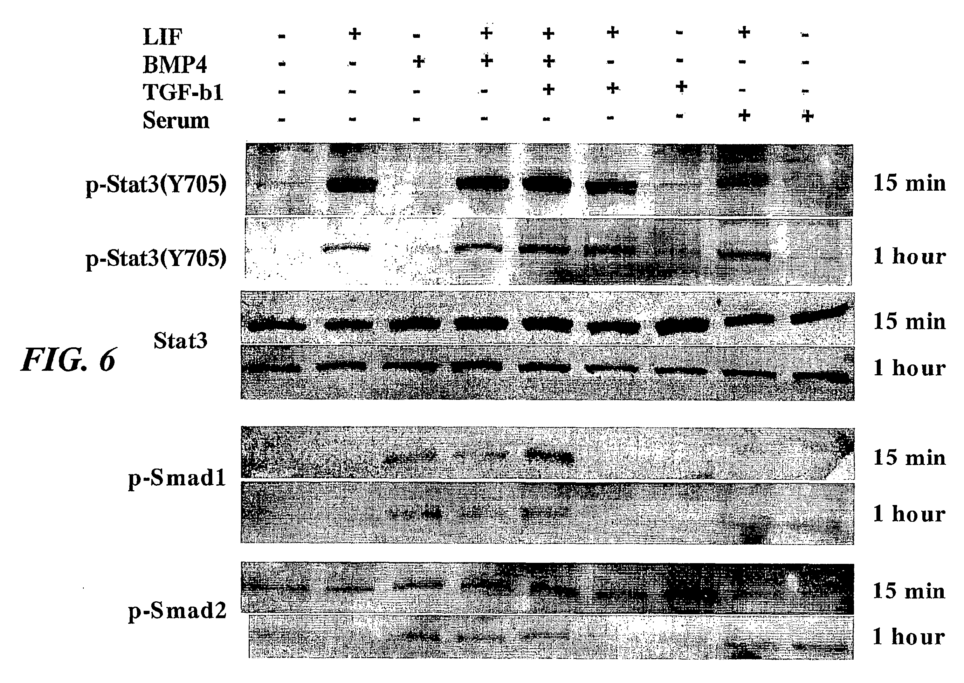

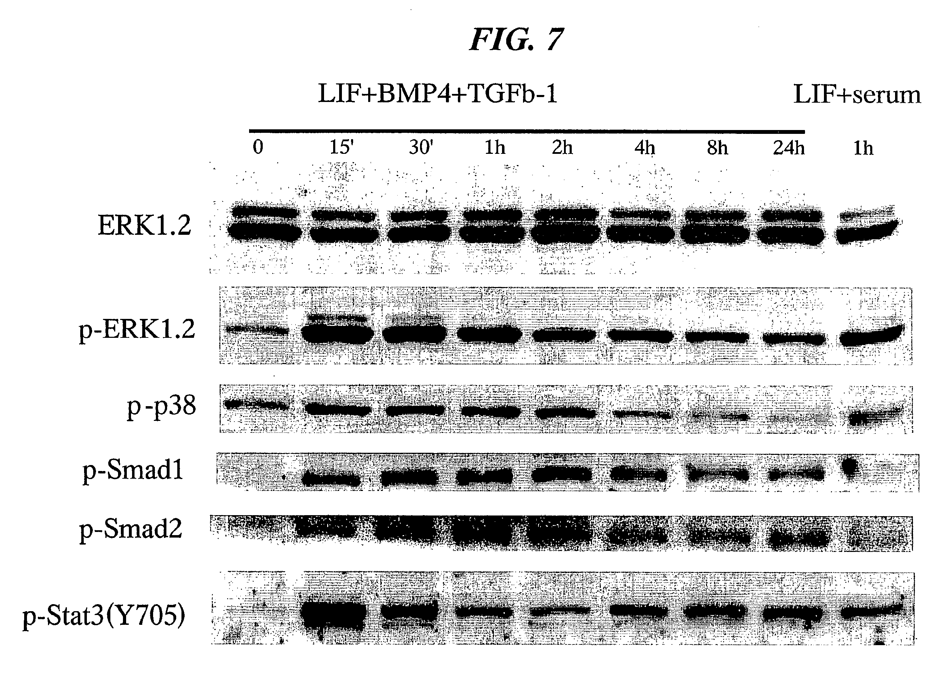

Administration of LIF and BMP4 was observed to lead to phosphorylation of SMADs, ERK and STAT3 (see Figs. 6 and 7) - demonstrating that there is activation of pathways downstream from the BMP and gp130 receptors.

### Example 8. Comparison of ES cells grown in serum-free and serum-containing medium.

We cultured Oct4GFP ES cells in 0.1% gelatin coated 6 well plates at a density of 4 x 10⁵ cells per well in the following media: N2B27 plus LIF, N2B27 plus LIF plus BMP4 and GMEM 10% FCS plus LIF. The results are shown in Table 1. Cell dissociation buffer was used to dissociate cells. After passage 5 the number of cells grown in N2B27 plus LIF was not sufficient to continue culture.

The invention thus provides medium and methods for self-renewal of ES cells of many species.

| **Comparison of ES cells grown in serum-free and serum-containing medium** | | | | | | |
|---|---|---|---|---|---|---|
| | **N2B27+LIF** | | **N2B27+LIF+BMP4** | | **GMEM/10%FCS+LIF** | |
| | Fold change | Oct4GFP% | Fold change | Oct4GFP% | Fold change | Oct4GFP% |
| P1 | 3.1±0.4 | 93.7±0.8 | 2.4±0.3 | 93.3±0.8 | 2.4±0.3 | 92.4±2.4 |
| P2 | 2.3±0.5 | 96.1±1.6 | 2.2t0.3 | 95.5±1.0 | 1.8±0.3 | 96.8±0.4 |
| P3 | 3.5±0.7 | 95.0±2.2 | 2.7±0.5 | 93.5±2.8 | 3.3±0.7 | 94.0±1.9 |
| P4 | 2.2±0.5 | 91.4±2.4 | 3.6±0.8 | 95.7±1.2 | 1.7±0.2 | 90.3±0.9 |
| P5 | 1.3±0.4 | 95.3±2.6 | 2.4±0.2 | 97.7±1.8 | 1.9±0.2 | 95.1±3.7 |
| P6 | | | 3.2±0.3 | 97.2±1.0 | 1.5±0.3 | 94.2±2.4 |
| P7 | | | 3.9±1.0 | 97.4±0.4 | 2.0±0.5 | 86.0±4.7 |
| P8 | | | 3.4±0.2 | 95.7±0.6 | 2.1±0.3 | 91.9±6.7 |

| | | | | | | |
|---|---|---|---|---|---|---|
| Oct4GIP ES cells were cultured in 0.1% gelatin-coated 6-well plates at a density of 4×10⁵ cells/well in the medium as indicated. Cell dissociation buffer was used to dissociate cells. After passage 5, the number of cells grown in N2B27+LIF was not enough for continuing culture. | | | | | | |

## Claims

1. Use of a combination of:-
(a) a bone morphogenic protein (BMP); and
(b) a cytokine acting through gp130,
for promoting self renewal of pluripotent cells in culture, wherein the pluripotent cells are not of human embryonic origin.

2. Use according to Claim 1 wherein the BMP is BMP4.

3. Use according to Claim 1 or 2, wherein the cytokine is LIF.

4. Use according to any of Claims 1 to 3 wherein the cytokine is soluble IL-6 in combination with soluble IL-6 receptor.

5. Use according to any of Claims 1 to 5 wherein said pluripotent cells are ES cells and are cultured in the presence of a FGF receptor inhibitor, wherein the FGF receptor inhibitor is SU5402 or PD173074.

6. Use according to Claim 5 wherein said ES cells are first cultured in the presence of the FGF receptor inhibitor and subsequently this inhibitor is withdrawn.

7. Use according to any of Claims 1 to 6 for culture of pluripotent cells selected from mammalian and avian pluripotent cells.

8. Use according to any of Claims 1 to 7 for culture of ES cells selected from mammalian and avian ES cells.

9. Use according to any of Claims 1 to 8 for culture of pluripotent stem cells selected from rodent, bovine, porcine, ovine and primate pluripotent stem cells.

10. Use according to any of Claims 1 to 9 for culture of ES cells selected from rodent, bovine, porcine, ovine and primate ES cells.

11. Use according to Claim 7 or 8 for culture of rat or mouse cells.

12. Use according to Claim 7 or 8 for culture of human cells.

13. Use according to any of Claims 1 to 12 wherein ES cells are cultured in medium that is free of serum, serum extract, feeder cells and feeder cell extract.

14. Use according to any of Claims 1 to 13 wherein ES cells are cultured in a fully defined medium.

15. A method of culture of non-human ES cells so as to promote ES cell self renewal, comprising maintaining the ES cells in medium containing:-
a. a BMP; and
b. a cytokine acting through gp130.

16. A method according to Claim 15 wherein the cytokine is LIF or IL-6 plus soluble IL-6 receptor.

17. A method according to Claim 15, wherein the medium further comprises an inhibitor of a FGF receptor which is SU5402 or PD173074

18. A method of culture of non-human ES cells, comprising:-
maintaining the ES cells in a pluripotent state in culture, optionally on feeders, in the presence of a cytokine acting though gp130 and serum or an extract of serum;
passaging the ES cells at least once;
withdrawing the serum or the serum extract from the medium and withdrawing the feeders if present, so that the medium is free of feeders, serum and serum extract; and
subsequently maintaining ES cells in a pluripotent state in the presence of
(h) a BMP; and
(i) a cytokine acting through gp130.

19. A method according to Claim 18, comprising culturing the ES cells in the presence of an inhibitor of an FGF receptor which is SU5402 or PD173074.

20. A method according to Claim 19 wherein the inhibitor is added to culture medium at around the time that serum or serum extract is withdrawn.

21. A method according to Claim 20 wherein at the same time as or subsequent to maintenance of the cells in the presence of a BMP, the inhibitor is withdrawn.

22. A method of obtaining a transfected population of non-human ES cells, comprising:-
transfecting ES cells with a construct encoding a selectable marker;
plating the ES cells;
culturing the ES cells in the presence of
(a) a BMP; and
(b)a cytokine acting through gp130; and
selecting for ES cells that express the selectable marker;

23. A method according to Claim 22 wherein the selectable marker encodes antibiotic resistance or a cell surface marker.

24. A method of culture of non-human ES cells, comprising:-
transferring an individual ES cell to a culture vessel; and
culturing the ES cell in the presence of
(a) a BMP; and
(b) a cytokine acting through gp130,
so as to obtain a clonal population of ES cells, all of which are progeny of the ES cell.

25. A method according to Claim 24 wherein the culture vessel is an individual well on a plate.

26. A method of directing differentiation of a non-human ES cell towards a neuroectodermal fate, comprising:- ,
maintaining the ES cell in the presence of:-
(a) a BMP; and
(b) a cytokine acting through gp130, and
withdrawing both (a) and (b) from the medium.

27. A method of directing differentiation of a non-human ES cell towards a non-neuroectodermal fate, comprising:-
maintaining the ES cell in the presence of
(a) a cytokine acting through gp130; and
(b) a BMP; and
withdrawing the cytokine whilst
(I) maintaining the BMP; and/or
(II) adding a further signalling molecule capable of directing differentiation.

28. A medium for self-renewal of ES cells, comprising:-
basal medium;
a BMP;
a cytokine acting through gp130; and
an iron transporter;
wherein the medium is free of serum or serum extract.

29. A medium according to Claim 28, further comprising insulin or insulin-like growth factor.

30. A medium according to Claim 28 or 29 wherein the medium is free of feeder cells or feeder cell extract.

31. An ES cell culture medium comprising:-
(a) a BMP;
(b) a cytokine acting through gp130; and
wherein the medium is free of serum and free of serum extract.

32. An ES cell culture medium comprising:-
(a) a BMP;
(b) a cytokine acting through gp130; and
wherein the medium is fully defined.

33. A medium according to Claim 31, further comprising an inhibitor of a FGF receptor which is SU5402 or PD173074.

34. A medium according to claims 29 to 31, wherein the cytokine acting through gp130 is LIF at a concentration of at least 10 U/ml.

35. A method of obtaining a differentiated cell comprising culturing an ES cell according to any of Claims 15 to 27 and allowing or causing an ES cell to differentiate, wherein the ES cell contains a selectable marker which is capable of differential expression in the desired differentiated cell compared with other cell-types, including pluripotent stem cells, whereby differential expression of the selectable marker results in preferential isolation and/or survival and/or division of the desired differentiated cells.

36. A method according to Claim 35 wherein the differentiated cell is a tissue stem cell or tissue progenitor cell.

37. A method according to Claim 36 wherein the differentiated cell is a terminally differentiated cell.

38. A method according to any of Claims 15 to 27 or 35 to 37 wherein the medium is a fully defined medium.

39. A method of obtaining differentiated cells, comprising:-
maintaining non-human ES cells in a medium comprising
(a) a BMP; and
(b) a cytokine acting through gp130 downstream signalling pathway;
removing one or both of (a) and (b) from the medium;
optionally, adding other signalling factors to the culture; and
thereby obtaining the desired differentiated cells.

## Patentansprüche

1. Verwendung einer Kombination aus:-
(a) einem morphogenetischen Knochenprotein (Bone Morphogenetic Protein: BMP); und
(b) einem Cytokin, das durch gp130 wirkt,
zur Förderung der Selbsterneuerung von pluripotenten Zellen in Kultur, wobei die pluripotenten Zellen nicht aus menschlichen Embryonen stammen.

2. Verwendung nach Anspruch 1, wobei das BMP BMP-4 ist.

3. Verwendung nach Anspruch 1 oder 2, wobei das Cytokin LIF ist.

4. Verwendung nach einem der Ansprüche 1 bis 3, wobei das Cytokin lösliches IL-6 in Kombination mit dem löslichen IL-6-Rezeptor ist.

5. Verwendung nach einem der Ansprüche 1 bis 4, wobei die pluripotenten Zellen ES-Zellen sind und in der Gegenwart von einem FGF-Rezeptor-Inhibitor kultiviert werden, wobei der FGF-Rezeptor-Inhibitor SU5402 oder PD173074 ist.

6. Verwendung nach Anspruch 5, wobei die ES-Zellen zuerst in der Gegenwart von dem FGF-Rezeptor-Inhibitor kultiviert sind und anschließend dieser Inhibitor entfernt wird.

7. Verwendung nach einem der Ansprüche 1 bis 6 zur Kultur von pluripotenten Zellen, die aus den pluripotenten Zellen von Säugern und Vögeln ausgewählt werden.

8. Verwendung nach einem der Ansprüche 1 bis 7 zur Kultur von ES-Zellen, die aus den ES-Zellen von Säugern und Vögeln ausgewählt werden.

9. Verwendung nach einem der Ansprüche 1 bis 8 zur Kultur von pluripotenten Stammzellen, die aus den pluripotenten Stammzellen von Nagern, Rindern, Schweinen, Schafen und Primaten ausgewählt werden.

10. Verwendung nach einem der Ansprüche 1 bis 9 zur Kultur von ES-Zellen, die aus den ES-Zellen von Nagern, Rindern, Schweinen, Schafen und Primaten ausgewählt werden.

11. Verwendung nach Anspruch 7 oder 8 zur Kultur von Ratten- oder Mäusezellen.

12. Verwendung nach Anspruch 7 oder 8 zur Kultur von menschlichen Zellen.

13. Verwendung nach einem der Ansprüche 1 bis 12, wobei die ES-Zellen in einem Medium kultiviert werden, das frei von Serum, Serumextrakt, Feederzellen und Extrakt von Feederzellen ist.

14. Verwendung nach einem der Ansprüche 1 bis 13, wobei die ES-Zellen in einem definierten synthetischen Medium kultiviert werden.

15. Verfahren zur Kultur von nicht-menschlichen ES-Zellen, um die Selbsterneuerung von ES-Zellen zu fördern, umfassend das Erhalten der ES-Zellen in einem Medium, das enthält:-
a. ein BMP; und
b. ein Cytokin, das durch gp130 wirkt,

16. Verfahren nach Anspruch 15, wobei das Cytokin LIF oder IL-6 plus löslichem IL-6-Rezeptor ist.

17. Verfahren nach Anspruch 15, wobei das Medium ferner einen Inhibitor von einem FGF-Rezeptor umfasst, welcher SU5402 oder PD173074 ist.

18. Verfahren zur Kultur von nicht-menschlichen ES-Zellen, umfassend:-
Erhalten der ES-Zellen in einem pluripotenten Zustand in der Kultur, wahlweise auf Feederzellen, in der Gegenwart von einem Cytokin, das durch gp130 wirkt, und Serum oder einem Extrakt von Serum;
Mindestens einmal Passagieren der ES-Zellen;
Entfernen von dem Serum oder dem Serumextrakt aus dem Medium und Entfernen der Feederzellen, falls vorhanden, derart, dass das Medium frei von Feederzellen, Serum und Serumextrakt ist; und
anschließendes Erhalten der ES-Zellen in einem pluripotenten Zustand in der Gegenwart von
(h) einem BMP; und
(i) einem Cytokin, das durch gp130 wirkt.

19. Verfahren nach Anspruch 18, wobei dieses das Kultivieren der ES-Zellen in der Gegenwart von einem Inhibitor von einem FGF-Rezeptor, welcher SU5402 oder PD173074 ist, umfasst.

20. Verfahren nach Anspruch 19, wobei der Inhibitor um den Zeitpunkt herum, wo das Serum oder der Serumextrakt entfernt werden, zu dem Kulturmedium hinzugefügt wird.

21. Verfahren nach Anspruch 20, wobei zur selben Zeit oder anschließend an die Erhaltung von den Zellen in der Gegenwart von BMP der Inhibitor entfernt wird.

22. Verfahren zur Erlangung einer transfizierten Population von nicht-menschlichen ES-Zellen, umfassend:-
Transfizieren von ES-Zellen mit einem Konstrukt, das einen auswählbaren Marker kodiert;
Ausplattieren der ES-Zellen;
Kultivieren der ES-Zellen in der Gegenwart von
(a) einem BMP; und
(b) einem Cytokin, das durch gp130 wirkt; und
Auswählen von den ES-Zellen, welche den auswählbaren Marker exprimieren.

23. Verfahren nach Anspruch 22, wobei der auswählbare Marker eine Antibiotika-Resistenz oder einen Zelloberflächen-Marker kodiert.

24. Verfahren zur Kultur von nicht-menschlichen ES-Zellen, das umfasst:-
Überführen von einer individuellen ES-Zelle in ein Kulturgefäß; und
Kultivieren der ES-Zelle in der Gegenwart von
(a) einem BMP; und
(b) einem Cytokin, das durch gp130 wirkt,
um so eine klonale Population von ES-Zellen zu erhalten, von denen alle Nachkommen von der ES-Zelle sind.

25. Verfahren nach Anspruch 24, wobei das Kulturgefäß eine einzelne Vertiefung in einer Platte ist.

26. Verfahren zum Steuern der Differenzierung von einer nicht-menschlichen ES-Zelle in Richtung auf eine neuroektodermale Entwicklung, welches umfasst:-
Erhaltung der ES-Zelle in der Gegenwart von:-
(a) einem BMP; und
(b) einem Cytokin, das durch gp130 wirkt, und Entfernen sowohl von (a) als auch von (b) aus dem Medium.

27. Verfahren zum Steuern der Differenzierung von einer nicht-menschlichen ES-Zelle in Richtung auf eine nicht-neuroektodermale Entwicklung, welches umfasst:-
Erhaltung der ES-Zelle in der Gegenwart von
(a) einem Cytokin, das durch gp130 wirkt; und
(b) einem BMP; und
Entfernen von dem Cytokin während
(I) dem Beibehalten von dem BMP; und/oder
(II) dem Hinzufügen von einem weiteren Signalmolekül, das in der Lage ist, die Differenzierung zu steuern.

28. Medium zur Selbsterneuerung der ES-Zellen, umfassend:- Basalmedium;
ein BMP;
ein Cytokin, das durch gp130 wirkt; und
ein Eisen-Transportprotein;
wobei das Medium frei von Serum oder Serumextrakt ist.

29. Medium nach Anspruch 28, das ferner Insulin oder Insulinartiger Wachstumsfaktor umfasst.

30. Medium nach Anspruch 28 oder 29, wobei das Medium frei von Feederzellen oder dem Extrakt von Feederzellen ist.

31. ES-Zellkulturmedium, das umfasst:-
(a) ein BMP;
(b) ein Cytokin, das durch gp130 wirkt; und
wobei das Medium frei von Serum oder Serumextrakt ist.

32. ES-Zellkulturmedium, das umfasst:-
(a) ein BMP;
(b) ein Cytokin, das durch gp130 wirkt, und wobei das Medium ein definiertes synthetisches Medium ist.

33. Medium nach Anspruch 31, wobei es ferner einen Inhibitor von einem FGF-Rezeptor umfasst, welcher SU5402 oder PD173074 ist.

34. Medium nach Anspruch 29 bis 31, wobei das Cytokin, das durch gp130 wirkt, LIF ist, mit einer Konzentration von mindestens 10 U/ml.

35. Verfahren zum Erlangen einer differenzierten Zelle, welches das Kultivieren einer ES-Zelle nach einem der Ansprüche 15 bis 27 und das Zulassen oder Auslösen der Differenzierung einer ES-Zelle umfasst, wobei die ES-Zelle einen auswählbaren Marker enthält, welcher gegenüber anderen Zelltypen zur differentiellen Expression in der erwünschten differenzierten Zelle, einschließlich pluripotenter Stammzellen, in der Lage ist, wobei die differentielle Expression von dem auswählbaren Marker in der bevorzugten Isolierung und/oder dem Überleben und/oder der Teilung von den erwünschten differenzierten Zellen resultiert.

36. Verfahren nach Anspruch 35, wobei die differenzierte Zelle eine Gewebestammzelle oder eine Gewebe-Vorläuferzelle ist.

37. Verfahren nach Anspruch 36, wobei die differenzierte Zelle eine terminal differenzierte Zelle ist.

38. Verfahren nach einem der Ansprüche 15 bis 27 oder 35 bis 37, wobei das Medium ein definiertes synthetisches Medium ist.

39. Verfahren zum Erlangen von differenzierten Zellen, das umfasst:-
Erhalten von nicht-menschlichen ES-Zellen in einem Medium, das umfasst:
(a) ein BMP; und
(b) ein Cytokin, das durch den gp130 nachgeschalteten Signaltransduktionsweg wirkt, und Entfernen von einem oder von beiden von (a) und (b) aus dem Medium;
wahlweise, das Hinzufügen anderer Signalfaktoren zu der Kultur und dadurch die erwünschten differenzierten Zellen zu erhalten.

## Revendications

1. Utilisation d'une combinaison :
(a) d'une protéine morphogénique osseuse (BMP) ; et
(b) d'une cytokine agissant par l'intermédiaire de la gp130,
pour favoriser l'auto-renouvellement de cellules pluripotentes en culture, où les cellules pluripotentes ne sont pas d'origine embryonnaire humaine.

2. Utilisation selon la revendication 1, où la BMP est la BMP4.

3. Utilisation selon la revendication 1 ou 2, où la cytokine est le LIF.

4. Utilisation selon l'une quelconque des revendications 1 à 3, où la cytokine est l'IL-6 soluble en combinaison avec le récepteur soluble de l'IL-6.

5. Utilisation selon l'une quelconque des revendications 1 à 5, où lesdites cellules pluripotentes sont des cellules SE et sont cultivées en présence d'un inhibiteur d'un récepteur du FGF, où l'inhibiteur d'un récepteur du FGF est le SU5402 ou le PD 173074.

6. Utilisation selon la revendication 5, où lesdites cellules SE sont d'abord cultivées en présence de l'inhibiteur d'un récepteur du FGF et ensuite cet inhibiteur est prélevé.

7. Utilisation selon l'une quelconque des revendications 1 à 6, pour la culture de cellules pluripotentes choisies parmi des cellules pluripotentes mammaliennes et aviaires.

8. Utilisation selon l'une quelconque des revendications 1 à 7, pour la culture de cellules SE choisies parmi des cellules SE mammaliennes et aviaires.

9. Utilisation selon l'une quelconque des revendications 1 à 8, pour la culture de cellules souches pluripotentes choisies parmi des cellules souches pluripotentes de rongeurs, bovines, porcines, ovines et de primates.

10. Utilisation selon l'une quelconque des revendications, 1 à 9 pour la culture de cellules SE choisies parmi des cellules SE de rongeurs, bovines, porcines, ovines et de primates.

11. Utilisation selon la revendication 7 ou 8, pour la culture de cellules de rat ou de souris.

12. Utilisation selon la revendication 7 ou 8, pour la culture de cellules humaines.

13. Utilisation selon l'une quelconque des revendications 1 à 12, où les cellules SE sont cultivées dans un milieu qui est dépourvu de sérum, d'extrait de sérum, de cellules nourricières et d'extrait de cellules nourricières.

14. Utilisation selon l'une quelconque des revendications 1 à 13, où les cellules SE sont cultivées dans un milieu totalement défini.

15. Procédé de culture de cellules SE non humaines de manière à favoriser l'auto-renouvellement des cellules SE, comprenant le maintien des cellules SE dans un milieu comprenant :
a. une BPM ; et
b. une cytokine agissant par l'intermédiaire de la gp130.

16. Procédé selon la revendication 15, dans lequel la cytokine est le LIF ou l'IL-6 plus un récepteur soluble de l'IL-6.

17. Procédé selon la revendication 15, dans lequel le milieu comprend en outre un inhibiteur d'un récepteur du FGF qui est le SU5402 ou le PD173074.

18. Procédé de culture de cellules SE non humaines, comprenant :
le maintien des cellules SE dans un état pluripotent en culture, éventuellement sur des cellules nourricières, en présence d'une cytokine agissant par l'intermédiaire de la gp130 et de sérum ou d'un extrait de sérum ;
le passage des cellules SE au moins une fois ;
le prélèvement du sérum ou de l'extrait de sérum à partir du milieu et le prélèvement des cellules nourricières, si présentes, de manière à ce que le milieu soit dépourvu de cellules nourricières, de sérum et d'extrait de sérum ; et
ensuite le maintien des cellules SE dans un état pluripotent en présence
(h) d'une BMP ; et
(i) d'une cytokine agissant par l'intermédiaire de la gp130.

19. Procédé selon la revendication 18, comprenant la culture des cellules SE en présence d'un inhibiteur d'un récepteur du FGF qui est le SU5402 ou le PD173074.

20. Procédé selon la revendication 19, dans lequel l'inhibiteur est ajouté au milieu de culture à peu près au moment lorsque le sérum ou l'extrait de sérum est prélevé.

21. Procédé selon la revendication 20, dans lequel en même temps que ou après le maintien des cellules en présence d'une BMP, l'inhibiteur est prélevé.

22. Procédé d'obtention d'une population transfectée de cellules SE non humaines comprenant :
la transfection des cellules SE avec un produit de construction codant pour un marqueur sélectionnable ;
le plaquage des cellules SE ;
la culture des cellules SE en présence
(a) d'une BMP ; et
(b) d'une cytokine agissant par l'intermédiaire de la gp130 ; et
la sélection des cellules SE qui expriment le marqueur sélectionnable.

23. Procédé selon la revendication 22, dans lequel le marqueur sélectionnable code pour une résistance à un antibiotique ou un marqueur de la surface cellulaire.

24. Procédé de culture de cellules SE non humaines, comprenant :
le transfert d'une cellule SE individuelle dans un récipient de culture ; et
la culture de la cellule SE en présence
(a) d'une BMP ; et
(b) d'une cytokine agissant par l'intermédiaire de la gp130,
de manière à obtenir une population clonale de cellules SE, dont la totalité est une descendance de la cellule SE.

25. Procédé selon la revendication 24, dans lequel le récipient de culture est un puits individuel sur une plaque.

26. Procédé de direction de la différenciation d'une cellule SE non humaine vers une destinée neuro-ectodermique, comprenant :
le maintien de la cellule SE en présence :
(a) d'une BMP ; et
(b) d'une cytokine agissant par l'intermédiaire de la gp 130, et
le prélèvement de, à la fois, (a) et (b) à partir du milieu.

27. Procédé de direction de la différenciation d'une cellule SE non humaine vers une destinée non neuro-ectodermique, comprenant :
le maintien de la cellule SE en présence
(a) d'une cytokine agissant par l'intermédiaire de la gp130 ; et
(b) d'une BMP ; et
le prélèvement de la cytokine tout en
(I) maintenant la BMP ; et/ou
(II) ajoutant une autre molécule de signalisation capable de diriger la différenciation.

28. Milieu pour l'auto-renouvellement des cellules SE, comprenant :
un milieu de base ;
une BMP ;
une cytokine agissant par l'intermédiaire de la gp130 ; et
un transporteur du fer ;
où le milieu est dépourvu de sérum ou d'extrait de sérum.

29. Milieu selon la revendication 28, comprenant en outre de l'insuline ou du facteur de croissance de type insulinique.

30. Milieu selon la revendication 28 ou 29, où le milieu est dépourvu de cellules nourricières ou d'extrait de cellules nourricières.

31. Milieu de culture de cellules SE comprenant :
(a) une BMP ;
(b) une cytokine agissant par l'intermédiaire de la gp130 ; et
où le milieu est dépourvu de sérum et dépourvu d'extrait de sérum.

32. Milieu de culture de cellules SE comprenant :
(a) une BMP ;
(b) une cytokine agissant par l'intermédiaire de la gp130 ; et
où le milieu est totalement défini.

33. Milieu selon la revendication 31, comprenant en outre un inhibiteur d'un récepteur du FGF qui est le SU5402 ou le PD173074.

34. Milieu selon les revendications 29 à 31, dans lequel la cytokine agissant par l'intermédiaire de la gp130 est le LIF à une concentration d'au moins 10 U/ml.

35. Procédé d'obtention d'une cellule différenciée comprenant les étapes consistant à cultiver une cellule SE selon l'une quelconque des revendications 15 à 27 et à permettre ou provoquer la différenciation d'une cellule SE, où la cellule SE contient un marqueur sélectionnable qui est capable d'expression différentielle dans la cellule différenciée souhaitée par comparaison à d'autres types cellulaires, y compris des cellules souches pluripotentes, moyennant quoi l'expression différentielle du marqueur sélectionnable entraîne l'isolement préférentiel et/ou la survie et/ou la division des cellules différenciées souhaitées.

36. Procédé selon la revendication 35, dans lequel la cellule différenciée est une cellule souche de tissu ou une cellule progéniteur de tissu.

37. Procédé selon la revendication 36, dans lequel la cellule différenciée est une cellule complètement différenciée.

38. Procédé selon l'une quelconque des revendications 15 à 27 ou 35 à 37, dans lequel le milieu est totalement défini.

39. Procédé d'obtention de cellules différenciées, comprenant :
le maintien de cellules SE non humaines dans un milieu comprenant
(a) une BMP ; et
(b) une cytokine agissant par l'intermédiaire de la gp130 en aval de la voie de signalisation ;
le prélèvement de l'un ou des deux de (a) et (b) à partir du milieu ;
éventuellement, l'addition d'autres facteurs de signalisation à la culture ; et
moyennant quoi l'obtention des cellules différenciées souhaitées.
